# EUROPEAN PATENT SPECIFICATION

(11) **EP 4 319 852 B1**
(45) Date of publication and mention of the grant of the patent: **30.10.2024**
(21) Application number: 22785790.1
(22) Date of filing: 15.09.2022
(51) Int. Cl.: A61M 25/04, A61M 25/09, A61M 25/10, A61B 17/00

(54) **A DEVICE FOR INSERTING A GUIDE WIRE INTO A BLOOD VESSEL**
VORRICHTUNG ZUM EINFÜHREN EINES FÜHRUNGSDRAHTES IN EIN BLUTGEFÄSS
DISPOSITIF POUR INTRODUIRE UN FIL DE GUIDAGE DANS UN VAISSEAU SANGUIN

(30) Priority: 20.09.2021 IT 202100024047
(43) Date of publication of application: 14.02.2024
(73) Proprietor: Vivheart S.r.l., 20121 Milano MI (IT)
(72) Inventor: MONTORFANO, Matteo, 20122 MILANO MI (IT)
(74) Representative: Barbaro, Gaetano
(86) International application number: PCT/IB2022/058699
(87) International publication number: WO 2023/042109

(56) References cited:
- WO-A1-2019/138334
- WO-A2-2008/027375
- US-A1- 2011 196 410

## Description

### TECHNICAL FIELD

The present disclosure relates in general to catheters and more in particular to a device for inserting a guidewire into a blood vessel, wherein the device is self-centering and may be inserted into an artery for directing a guidewire through a heart valve.

### BACKGROUND OF THE INVENTION

Percutaneous valve replacement (Transcatheter Aortic Valve Replacement or more briefly TAVR) is a technique performed by inserting a catheter containing a guidewire, which is used as a guide for the implantation of a cardiac valve prosthesis. The catheter is threaded through the femoral artery and pushed up to the proximity of the defective heart valve or through the chest, for exiting the guidewire contained in the catheter near the cardiac valve so that it passes throughout it. Once the guidewire has passed throughout the heart valve, it is used to guide a valve prosthesis to the heart, where it must be positioned.

A difficulty related to this type of technique consists in the fact that the operator must be particularly skilled in order to quickly insert the guidewire through the faulty valve. The imaging equipment currently used in operating rooms only allow a two-dimensional view and not a three-dimensional view of the guidewire approaching the defective heart valve. As a consequence, from the two-dimensional image the operator may have the wrong impression of correctly directing the guidewire to the center of the heart valve, which is also narrow and degenerated, crooked, when instead the apical end of the guidewire is going to abut against the walls of the aorta.

In any case, even if a second apparatus for images arranged so as to provide a different view were available, there would be the problem of positioning and properly orienting a catheter while pushing forward the guidewire. Moreover, the guidewire is made of yielding material so as not to damage the tissues with which it comes into contact, so it bends easily even when it should remain straight to pass through the defective valve. This problem is even more pronounced in the so-called Valve-In-Valve technique (or more briefly VIV), in which a defective valve prosthesis is not completely removed, but is used as a frame in which a new percutaneous heart valve prosthesis is anchored. This new technique prevents the patient from the trauma of removing the old prosthetic valve, which remains attached to the heart tissue while the new valve prosthesis is attached to the old. Nevertheless this is critical because the guidewire, if pushed against the damaged portions of the old heart valve prosthesis, may cause the detachment of a part of it.

The document US2014/0207179 discloses a device, shown in Figure 1, for centering a catheter 190 in which to pass a surgical guide wire 192. This anterior device has a plurality of thread-like elements 186 of equal length having ends fixed to a distal element 188 and to a proximal element 194. The thread-like elements 186, having thermal memory, bend and move radially away over the internal catheter 190 the distal element 188 retracting it. If the device is inserted in a substantially cylindrical portion of a blood vessel, the thread-like elements 186 abut against the blood vessel wall in a practically symmetrical manner, so that the internal catheter 190 is substantially placed at the center of the blood vessel.

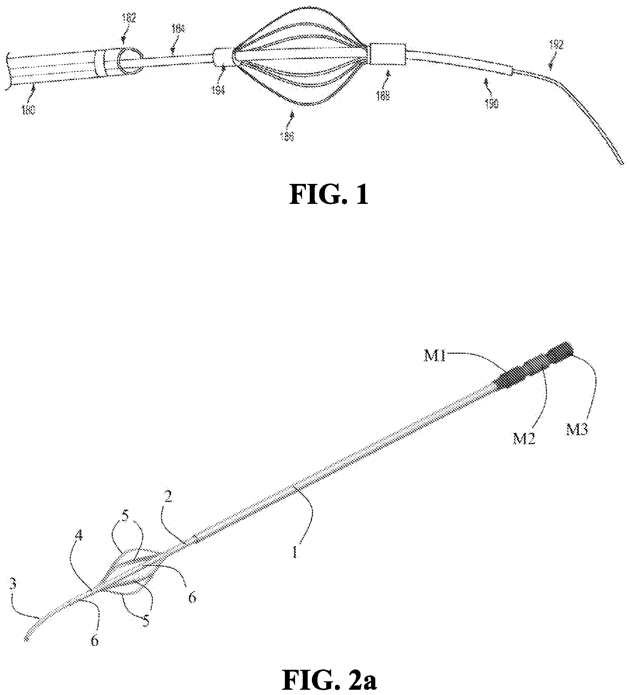

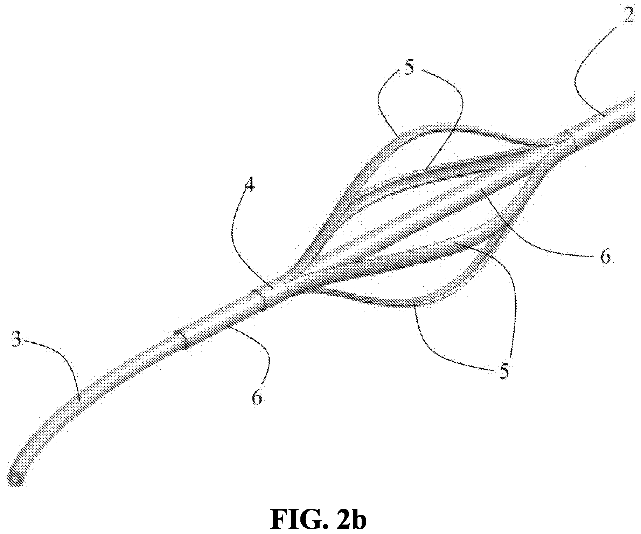

The document WO2019138334 discloses a device, shown in figure 2a and in the detail view of figure 2b, comprising three catheters 1, 2, 3, with different characteristics, one inserted inside the other, to direct a guiding wire through a blood vessel and / or heart valve. Like the device of figure 1, it has thread-like elements 5 of equal length having ends fixed to a distal element 4 and to a proximal element 2. The distal element slides over a tubular portion 6 of the intermediate catheter 2. A more internal catheter 3, intended to conduct a surgical guide wire, tends to spontaneously bend in a more accentuated manner the more it protrudes from the tubular portion 6 of the intermediate catheter 2. An outermost catheter 1 is arranged so that the intermediate catheter 2 and the more internal 3 can be contained therein, so that the thread-like elements 5 can expand freely, exiting the outermost catheter 1 in proximity to the point where a surgical intervention is to be performed.

A drawback of this type of device is that, when inserted into a blood vessel and opened, the thread-like elements 186, 5 tend to flex along the circumferential direction as soon as they touch the blood vessel walls and move along the circumferential direction. This unexpected effect occurs in particular in large blood vessels such as the aorta. Consequently, often the catheter 190, 6, over which the distal element 188, 4 slides, is not immediately positioned in the desired way, for example in correspondence with the heart valves, so that the surgeon can only retract the thread-like elements 186, 5 into the external catheter 180, 1, and let them come out again until they are positioned in the desired way.

US2011/196410, upon the disclosure of which the preamble of claim 1 is drafted, discloses an endoluminal device including at least a control element connected to a surrounding sheath and an elastic bias section to control changes to a bias force formed between the control element and the sheath. By applying an external force at a proximal end of the device, the shape can change between varying degrees of deformed shapes and an undeformed shape.

WO2008/027375 discloses a gastrostomy or jejunal device for extended insertion into an abdomen of a patient.

### SUMMARY

An object of the present disclosure is to provide a device for inserting a guide wire suitable for heart valve prostheses into a blood vessel, defined in the attached claim 1, which solves at least partially the aforementioned drawbacks.

Tests carried out by the applicant with different prototypes have shown that it is easier to counteract unwanted displacements of the thread-like elements if the distal portion, to which the thread-like elements are connected, is fixed - for example by welding or gluing or other valid joining method - to the internal catheter and the internal catheter has a handle portion which allows the surgeon, by pulling it, to modulate the folding of the thread-like elements, around the longitudinal axis of the catheter. Furthermore, thanks to the fact that it is possible to shorten the distal portion of the external catheter with respect to the longitudinal axis, by withdrawing the internal catheter, it has been noted that it is particularly convenient to make the thread-like elements by means of a first plurality of notches and a second plurality of staggered notches between them along the longitudinal direction, carried out on a median portion of the external catheter, in order to quickly and firmly position the thread-like elements against irregular walls of a blood vessel.

Preferred embodiments are defined in the dependent claims.

### BRIEF DESCRIPTION OF THE DRAWINGS

Figure 1 shows a prior device for positioning a catheter carrying a surgical guide wire.
Figure 2a is an overall view of another prior device for positioning a catheter carrying a surgical guide wire.
Figure 2b is a detail view of the prior device of Figure 2a.
Figure 3 shows profile, top and detail views of an exemplary embodiment of this disclosure of a guide wire insertion device suitable for valve prostheses, in an extended configuration.
Figure 4 shows profile, top and detail views of the device of Figure 3 in an expanded configuration.
Figures 5a and 5b show in sequence how the thread-like elements fold / unfold by pulling / pushing a handle portion of the internal catheter of the device of Figures 3 and 4.
Figure 6 is a detail view of the thread-like elements of the device of Figures 3 and 4 folded radially outwards.
Figures 7a-7d show a triangular reinforcement portion of the thread-like elements of figure 6.

### DETAILED DESCRIPTION OF EXAMPLARY EMBODIMENTS

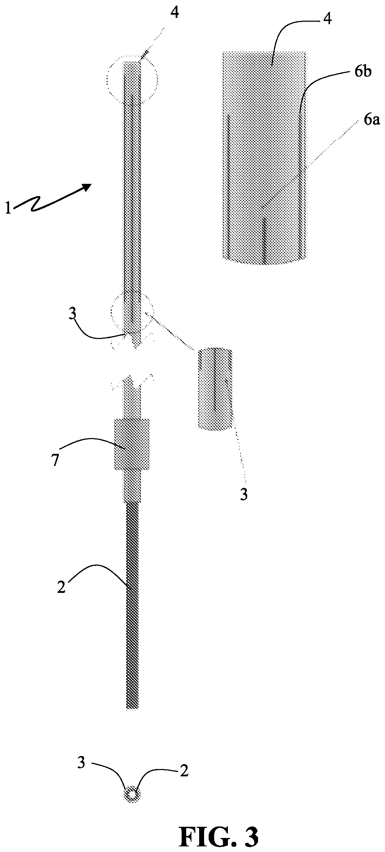

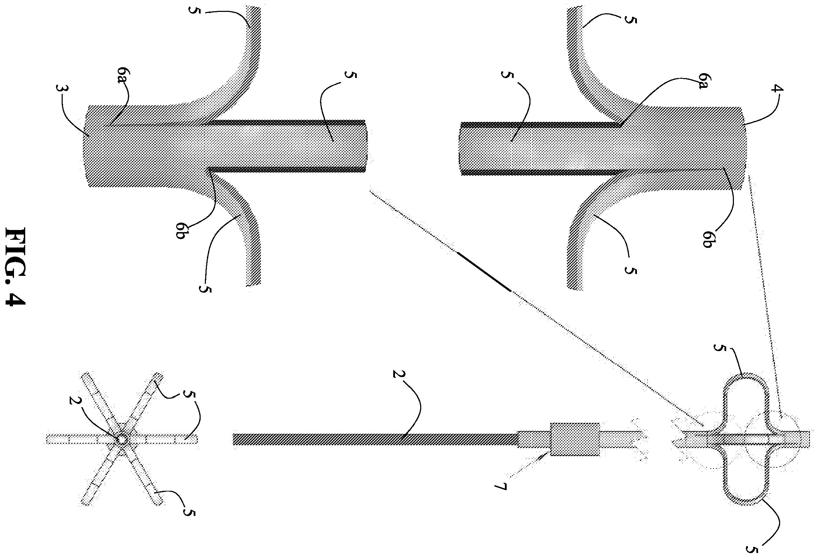

Characteristic features of the device of the present disclosure will be illustrated for simplicity with reference to the embodiment illustrated in Figures 3 and 4, but the skilled technician will recognize that the same observations hold, *mutatis mutandis*, for all the embodiments defined by the attached claims.

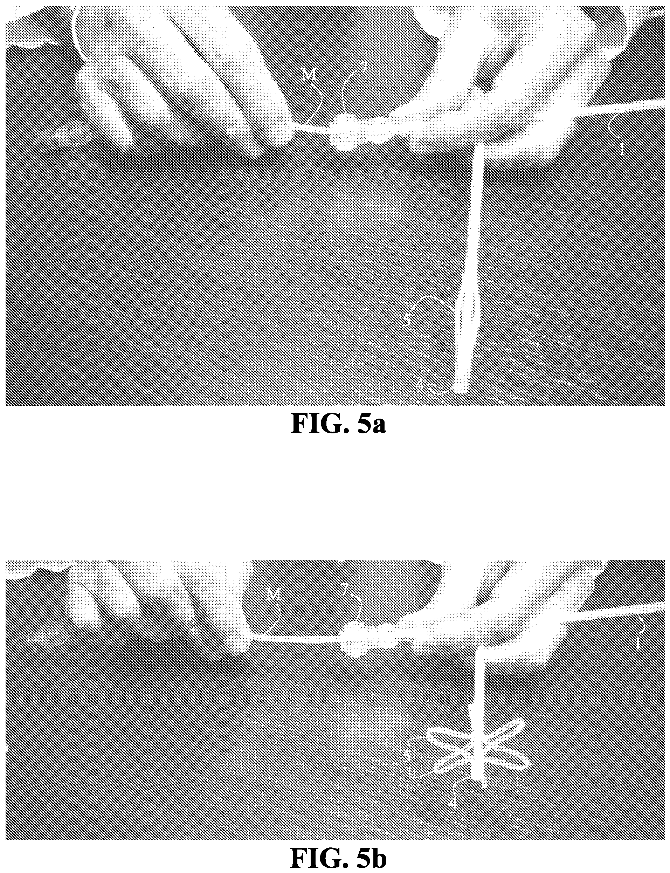

Generally, a device according to the present disclosure for inserting a guidewire suitable, for example, for heart valve prostheses in a blood vessel, comprising an external catheter 1 having a wall defining a longitudinal axis and a circumferential direction, and an internal catheter 2 inserted into the external catheter 1, having a handle portion M, shown in Figures 5a and 5b, configured to allow a user to advance or retract the internal catheter 2 along the longitudinal axis. The external catheter 1 defines an anchoring element to the internal walls of a blood vessel and comprises:
- a distal annular portion 4 of the wall of the external catheter 1, fixed to the internal catheter 2 so as to move integrally with the internal catheter 2 when it is advanced or retracted,
- a proximal annular portion 3 of the wall of the external catheter 1, sliding on the internal catheter 2, wherein the wall of the external catheter 1 does not have any interruption in correspondence with the distal annular portion 4 and with the proximal annular portion 3,
- a median annular portion of the wall of the external catheter 1, sliding on the internal catheter 2, comprising at least a first plurality of notches directed according to the longitudinal axis and having a first length, a second plurality of notches directed according to the longitudinal axis and having a second length, in which all the notches divide the wall of the median annular portion of the external catheter 1 and define between them a plurality of threadlike elements 5 suspended above the internal catheter 2.

The threadlike elements 5 allow a self-centering / spacing of the device to the internal walls of a blood vessel because they are configured to:
- folding away radially with respect to the internal catheter 2 when the handle portion M is pulled, causing the internal catheter 2 to retract along the longitudinal axis with respect to the external catheter 1, as shown in figure 5b, abutting against the internal walls of the blood vessel in opposite points, counteracting displacements of the internal catheter 2 transversely to the longitudinal axis,
- extending along the longitudinal axis when the handle portion M is pushed by advancing the internal catheter 2 along the longitudinal axis with respect to the external catheter 1.

A characteristic of the device of the present disclosure is therefore the fact that it allows the thread-like elements 5 to be folded / stretched through the internal catheter 2 with respect to the external catheter 1 by dragging with it the distal annular portion 4 of the external catheter 1, allowing them to abut / space themselves as desired against the inner walls of the blood vessel.

In the device of the present disclosure the notches of the first plurality of notches have respective ends 6a misaligned along the longitudinal axisto respective ends 6b of the notches of the second plurality of notches, so that the notches of the first plurality of notches are offset along the longitudinal axis with respect to the notches of the second plurality of notches.

This feature, combined with the possibility of gradually flexing the thread-like elements 5 (due to the fact that the distal annular portion 4 is fixed to the internal catheter 2), allows to realize devices for inserting a surgical guide wire which guarantees a good positioning of the internal catheter 2 even if the blood vessel has an irregular shape, and also other functions not allowed in prior devices.

According to one aspect, the notches of the first plurality have the same length as the notches of the second plurality.

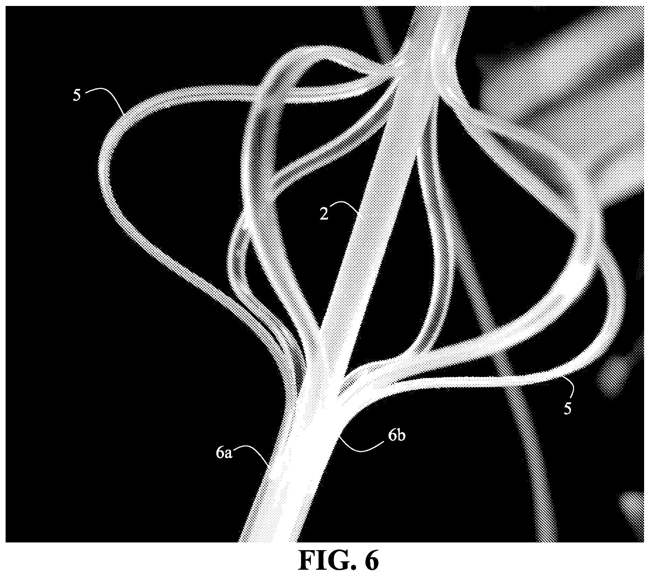

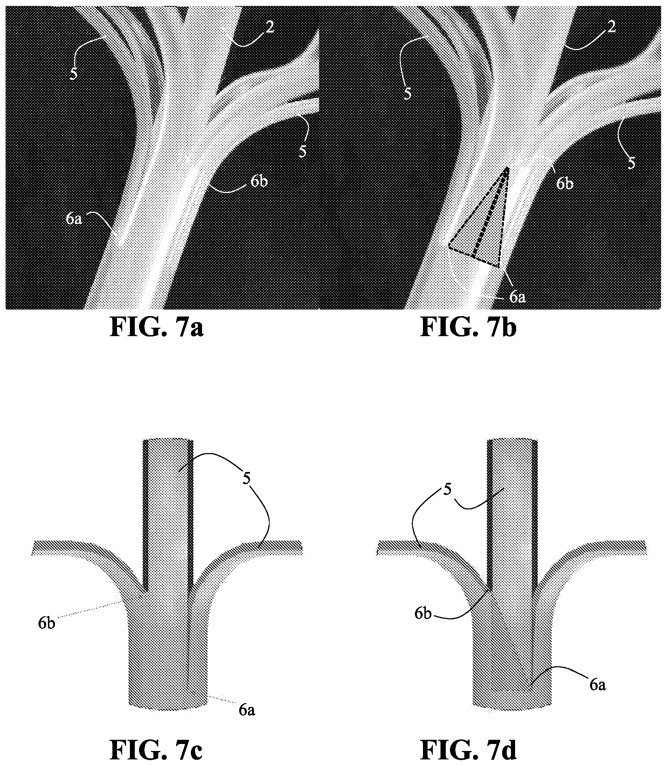

According to an optional aspect, to give greater resistance to the thread-like elements 5 to resist unwanted displacements in the circumferential direction,the notches of the first plurality of notches are orderly alternated with the notches of the second plurality of notches along the circumferential direction of the median portion of the wall of the external catheter 1, as shown in figures from 3 to 7d so that each threadlike element of the threadlike elements 5 is defined by a notch of the first plurality of notches and by a notch of the second plurality of notches. Figures 6, 7a and 7c show the thread-like elements 5 radially bent outwards and the triangular reinforcement portions, highlighted in figures 7b and 7d, which counteract the unwanted twisting of the thread-like elements 5 when they rest against the irregular internal walls of a blood vessel.

According to an optional aspect, as shown in figures 3 and 4, the notches of the first plurality of notches have their respective ends 6a aligned with each other along the circumferential direction of the median portion of the wall of the external catheter 1, and the notches of the second plurality of notches have their respective ends 6b aligned with each other along the circumferential direction of the median portion of the wall of the external catheter 1, so that all the thread-like elements 5 have the same triangular reinforcing portions.

According to one optional aspect, as shown in figures 3 and 4, the device may be equipped with a hemostatic valve 7 fixed to the external catheter 1, configured so that the internal catheter 2 slides tightly therethrough along the longitudinal axis. According to one aspect, the device of the present disclosure may comprise also a guiding catheter (not shown), preferably preformed, slidably inserted in said internal catheter 2, so as to further direct the guide wire into a blood vessel in proximity of a heart valve.

According to one aspect, the threadlike elements 5 are made of a biocompatible material suitable to be put into contact with human tissues.

According to an aspect of the present disclosure, the thread-like elements 5 can very simply be made by making the first plurality of notches and the second plurality of notches on a common catheter for surgical operations. For example, these notches can be made by machine using suitable blades, or they can be defined by laser cutting. According to an aspect of the present disclosure, the distal portion 4, to which the thread-like elements 5 are connected, can be fixed to the internal catheter 2 by welding or gluing or other valid method of mechanical joining.

The present invention has been described so far with reference to preferred embodiments. It is understood that there could be other embodiments which refer to the same inventive concept defined by the scope of the following claims.

## Claims

1. A device for inserting a guide wire suitable for heart valve prostheses into a blood vessel, comprising:
an external catheter (1) having a wall defining a longitudinal axis and a circumferential direction,
an internal catheter (2) inserted into said external catheter (1), having a handle portion (M) configured to allow a user to advance or retract said internal catheter (2) along said longitudinal axis,
wherein said external catheter (1) defines an anchoring element to the internal walls of a blood vessel,
said anchoring element comprising:
- a distal annular portion (4) of said wall of the external catheter (1), fixed to said internal catheter (2) so as to move integrally with said internal catheter (2) when it is advanced or retracted,
- a proximal annular portion (3) of said wall of the external catheter (1), slidable on said
internal catheter (2), wherein the wall of the external catheter (1) does not have any interruption in correspondence with said distal annular portion (4) and with said proximal annular portion (3),
- a median annular portion of said wall of the external catheter (1), slidable on said
internal catheter (2), comprising at least a first plurality of notches directed according to said longitudinal axis and having a first length, a second plurality of notches directed according to said longitudinal axis and having a second length, in which all said notches divide the wall of the median annular portion of the external catheter (1) and define between them a plurality of threadlike elements (5) suspended above said internal catheter (2);
wherein said threadlike elements (5) are configured for:
- folding away radially with respect to said internal catheter (2) when said handle portion (M) is pulled, causing the internal catheter (2) to retract along said longitudinal axis with respect to the external catheter (1), abutting against said internal walls of the blood vessel in opposite points, counteracting displacements of the internal catheter (2) transversely to said longitudinal axis,
- extending along the longitudinal axis when said handle portion (M) is pushed by advancing the internal catheter (2) along said longitudinal axis with respect to the external catheter (1);
wherein said notches of the first plurality of notches have respective ends (6a);
**characterized in that** said respective ends (6a) are misaligned along said longitudinal axis to respective ends (6b) of the notches of the second plurality of notches, so that the notches of the first plurality of notches are offset along the longitudinal axis with respect to the notches of the second plurality of notches.

2. The device according to claim 1, wherein said second length is equal to said first length.

3. The device according to one of the preceding claims, wherein the notches of said first plurality of notches are orderly alternated with the notches of said second plurality of notches along said circumferential direction of the median portion of the wall of the external catheter (1), so that each threadlike element of said threadlike elements (5) is defined by a notch of the first plurality of notches and by a notch of the second plurality of notches.

4. The device according to one of the preceding claims, in which the notches of said first plurality of notches have their respective ends (6a) aligned with each other along said circumferential direction of the median portion of the wall of the external catheter (1), and the notches of said second plurality of notches have their respective ends (6b) aligned with each other along said circumferential direction of the median portion of the wall of the external catheter (1).

5. The device according to one of the preceding claims, comprising a guide wire for heart valve prostheses inserted in said internal catheter (2).

6. The device according to one of the preceding claims, comprising a preformed guiding catheter slidably inserted in said internal catheter (2).

7. The device according to one of the preceding claims, in which said threadlike elements are made of a biocompatible material.

8. The device according to one of the preceding claims, further comprising a hemostatic valve (7) fixed to said external catheter (1) and configured so that said internal catheter (2) slides tightly therethrough along said longitudinal axis.

## Patentansprüche

1. Vorrichtung zum Einführen eines Führungsdrahtes, der für Herzklappenprothesen geeignet ist, in ein Blutgefäß, umfassend:
einen Außenkatheter (1), der eine Wand aufweist, die eine Längsachse und eine Umfangsrichtung definiert,
einen Innenkatheter (2), der in den Außenkatheter (1) eingeführt ist, der einen Griffabschnitt (M) aufweist, der konfiguriert ist, um einem Benutzer zu ermöglichen, den Innenkatheter (2) entlang der Längsachse vorzuschieben oder zurückzuziehen,
wobei der Außenkatheter (1) ein Verankerungselement an den Innenwänden eines Blutgefäßes definiert,
das Verankerungselement umfassend:
- einen distalen ringförmigen Abschnitt (4) der Wand des Außenkatheters (1), der an dem Innenkatheter (2) befestigt ist, um sich mit dem Innenkatheter (2) einstückig zu bewegen, wenn er vorgeschoben oder zurückgezogen wird,
- einen proximalen ringförmigen Abschnitt (3) der Wand des Außenkatheters (1), der auf dem Innenkatheter (2) verschiebbar ist, wobei die Wand des Außenkatheters (1) entsprechend dem distalen ringförmigen Abschnitt (4) und dem proximalen ringförmigen Abschnitt (3) keine Unterbrechung aufweist,
- einen mittleren ringförmigen Abschnitt der Wand des Außenkatheters (1), der auf dem Innenkatheter (2) verschiebbar ist, umfassend mindestens eine erste Vielzahl von Kerben, die gemäß der Längsachse gerichtet sind und eine erste Länge aufweisen, eine zweite Vielzahl von Kerben, die gemäß der Längsachse gerichtet sind und eine zweite Länge aufweisen, wobei alle Kerben die Wand des mittleren ringförmigen Abschnitts des Außenkatheters (1) unterteilen und zwischen sich eine Vielzahl fadenförmiger Elemente (5) definieren, die über dem Innenkatheter (2) aufgehängt sind;
wobei die fadenförmigen Elemente (5) konfiguriert sind zum:
- radialen Wegklappen in Bezug auf den Innenkatheter (2), wenn an dem Griffabschnitt (M) gezogen wird, wodurch veranlasst wird, dass sich der Innenkatheter (2) entlang der Längsachse in Bezug auf den Außenkatheter (1) zurückzieht, Anliegen an den Innenwänden des Blutgefäßes an entgegengesetzten Punkten, Entgegenwirken von Verlagerungen des Innenkatheters (2) quer zu der Längsachse,
- Erstrecken entlang der Längsachse, wenn der Griffabschnitt (M) durch Vorschieben des Innenkatheters (2) entlang der Längsachse in Bezug auf den Außenkatheter (1) geschoben wird;
wobei die Kerben der ersten Vielzahl von Kerben jeweilige Enden (6a) aufweisen;
**dadurch gekennzeichnet, dass die** jeweiligen Enden (6a) entlang der Längsachse mit den jeweiligen Enden (6b) der Kerben der zweiten Vielzahl von Kerben falsch ausgerichtet sind, sodass die Kerben der ersten Vielzahl von Kerben entlang der Längsachse in Bezug auf die Kerben der zweiten Vielzahl von Kerben versetzt sind.

2. Vorrichtung nach Anspruch 1, wobei die zweite Länge gleich der ersten Länge ist.

3. Vorrichtung nach einem der vorstehenden Ansprüche, wobei die Kerben der ersten Vielzahl von Kerben sich mit den Kerben der zweiten Vielzahl von Kerben entlang der Umfangsrichtung des Mittelabschnitts der Wand des Außenkatheters (1) ordnungsgemäß abwechseln, sodass jedes fadenförmige Element der fadenförmigen Elemente (5) durch eine Kerbe der ersten Vielzahl von Kerben und durch eine Kerbe der zweiten Vielzahl von Kerben definiert ist.

4. Vorrichtung nach einem der vorstehenden Ansprüche, bei der die jeweiligen Enden (6a) der Kerben der ersten Vielzahl von Kerben entlang der Umfangsrichtung des Mittelabschnitts der Wand des Außenkatheters (1) aufeinander ausgerichtet sind und die jeweiligen Enden (6b) der Kerben der zweiten Vielzahl von Kerben entlang der Umfangsrichtung des Mittelabschnitts der Wand des Außenkatheters (1) aufeinander ausgerichtet sind.

5. Vorrichtung nach einem der vorstehenden Ansprüche, umfassend einen Führungsdraht für Herzklappenprothesen, der in den Innenkatheter (2) eingeführt ist.

6. Vorrichtung nach einem der vorstehenden Ansprüche, umfassend einen vorgeformten Führungskatheter, der in den Innenkatheter (2) verschiebbar eingeführt ist.

7. Vorrichtung nach einem der vorstehenden Ansprüche, wobei die fadenförmigen Elemente aus einem biokompatiblen Material hergestellt sind.

8. Vorrichtung nach einem der vorstehenden Ansprüche, ferner umfassend ein hämostatisches Ventil (7), das an dem Außenkatheter (1) befestigt und konfiguriert ist, sodass sich der Innenkatheter (2) entlang der Längsachse dicht dahindurch verschiebt.

## Revendications

1. Dispositif permettant d'insérer un fil guide approprié pour des prothèses de valves cardiaques dans un vaisseau sanguin, comprenant :
un cathéter externe (1) ayant une paroi définissant un axe longitudinal et une direction circonférentielle,
un cathéter interne (2) inséré dans ledit cathéter externe (1), ayant une partie de poignée (M) conçue pour permettre à un utilisateur d'avancer ou de rétracter ledit cathéter interne (2) le long dudit axe longitudinal,
dans lequel ledit cathéter externe (1) définit un élément d'ancrage aux parois internes d'un vaisseau sanguin,
ledit élément d'ancrage comprenant :
- une partie annulaire distale (4) de ladite paroi du cathéter externe (1), fixée audit cathéter interne (2) de façon à se déplacer d'un seul tenant avec ledit cathéter interne (2) lorsqu'il est avancé ou rétracté,
- une partie annulaire proximale (3) de ladite paroi du cathéter externe (1), pouvant coulisser sur ledit cathéter interne (2), dans lequel la paroi du cathéter externe (1) n'a aucune interruption en correspondance avec ladite partie annulaire distale (4) et avec ladite partie annulaire proximale (3),
- une partie annulaire médiane de ladite paroi du cathéter externe (1), pouvant coulisser sur ledit cathéter interne (2), comprenant au moins une première pluralité d'encoches dirigées selon ledit axe longitudinal et ayant une première longueur, une seconde pluralité d'encoches dirigées selon ledit axe longitudinal et ayant une seconde longueur, où toutes lesdites encoches divisent la paroi de la partie annulaire médiane du cathéter externe (1) et définissent entre elles une pluralité d'éléments filiformes (5) suspendus au-dessus dudit cathéter interne (2) ;
dans lequel lesdits éléments filiformes (5) sont conçus pour :
- se replier de façon radiale par rapport audit cathéter interne (2) lorsque ladite partie de poignée (M) est tirée, faisant en sorte que le cathéter interne (2) se rétracte le long dudit axe longitudinal par rapport au cathéter externe (1), venant en butée contre lesdites parois internes du vaisseau sanguin en des points à l'opposé, contrebalançant les déplacements du cathéter interne (2) transversalement audit axe longitudinal,
- s'étendre le long de l'axe longitudinal lorsque ladite partie de poignée (M) est poussée en faisant avancer le cathéter interne (2) le long dudit axe longitudinal par rapport au cathéter externe (1) ;
dans lequel lesdites encoches de la première pluralité d'encoches ont des extrémités respectives (6a) ;
**caractérisé en ce que** lesdites extrémités respectives (6a) sont désalignées le long dudit axe longitudinal aux extrémités respectives (6b) des encoches de la seconde pluralité d'encoches, de sorte que les encoches de la première pluralité d'encoches sont décalées le long de l'axe longitudinal par rapport aux encoches de la seconde pluralité d'encoches.

2. Dispositif selon la revendication 1, dans lequel la seconde longueur est égale à ladite première longueur.

3. Dispositif selon l'une des revendications précédentes, dans lequel les encoches de ladite première pluralité d'encoches sont alternées de manière ordonnée avec les encoches de ladite seconde pluralité d'encoches le long de ladite direction circonférentielle de la partie médiane de la paroi du cathéter externe (1), de sorte que chaque élément filiforme desdits éléments filiformes (5) est défini par une encoche de la première pluralité d'encoches et par une encoche de la seconde pluralité d'encoches.

4. Dispositif selon l'une des revendications précédentes, où les encoches de ladite première pluralité d'encoches ont leurs extrémités respectives (6a) alignées les unes avec les autres le long de ladite direction circonférentielle de la partie médiane de la paroi du cathéter externe (1), et les encoches de ladite seconde pluralité d'encoches ont leurs extrémités respectives (6b) alignées les unes avec les autres le long de ladite direction circonférentielle de la partie médiane de la paroi du cathéter externe (1).

5. Dispositif selon l'une des revendications précédentes, comprenant un fil guide pour des prothèses de valves cardiaques inséré dans ledit cathéter interne (2).

6. Dispositif selon l'une des revendications précédentes, comprenant un cathéter de guidage préformé inséré de manière coulissante dans ledit cathéter interne (2).

7. Dispositif selon l'une des revendications précédentes, où lesdits éléments filiformes sont constitués d'un matériau biocompatible.

8. Dispositif selon l'une des revendications précédentes, comprenant en outre une valve hémostatique (7) fixée audit cathéter externe (1) et conçue de sorte que ledit cathéter interne (2) glisse fermement à travers celle-ci le long dudit axe longitudinal.
